# EUROPEAN PATENT SPECIFICATION

(11) **EP 4 522 635 B1**
(45) Date of publication and mention of the grant of the patent: **01.07.2026**
(21) Application number: 23726045.0
(22) Date of filing: 10.05.2023
(51) Int. Cl.: C07K 14/415, A61P 31/04, A61P 31/10, A61K 38/16, A01N 37/00, A01N 65/08

(54) **PEPTIDE HAVING ANTIMICROBIAL ACTIVITY**
PEPTIDE MIT ANTIMIKROBIELLER WIRKUNG
PEPTIDE AYANT UNE ACTIVITÉ ANTIMICROBIENNE

(30) Priority: 10.05.2022 EP 22172627
(43) Date of publication of application: 19.03.2025
(73) Proprietor: Cabosse Naturals NV, 1500 Halle (BE)
(72) Inventor: KOPP, Gabi, 1500 Halle (BE); BERNAERT, Herwig, 1500 Halle (BE); ULLRICH, Matthias, 28759 Bremen (DE); KUHNERT, Nikolai, 28759 Bremen (DE); KUMARI, Neha, 01454 Radeberg (DE)
(74) Representative: Brantsandpatents bv
(86) International application number: PCT/EP2023/062398
(87) International publication number: WO 2023/217848

(56) References cited:
- WO-A1-98/27805
- JOHN P. MARCUS ET AL: "Peptide Fragments From Plant Vicilins Expressed in Escherichia Coli Exhibit Antimicrobial Activity In Vitro", PLANT MOLECULAR BIOLOGY REPORTER., vol. 26, no. 2, 1 April 2008 (2008-04-01), NL, pages 75 - 87, XP055592751, ISSN: 0735-9640, DOI: 10.1007/s11105-008-0024-9

## Description

### FIELD OF THE INVENTION

The invention pertains to the technical field of antimicrobial peptides, more particularly natural plant-derived peptides, which exhibit antimicrobial activity against various bacterial and fungal strains. In particular, the present invention relates to a natural, recombinant, or synthetic peptide derived from the plant vicilin protein. The invention further relates to the use of the peptide for the treatment of bacterial and fungal infections in plants, animals, and humans.

### BACKGROUND

Bacterial antibiotic resistance is a significant issue faced by the food and agricultural industries, the medical and veterinary professions, and others. The potential for transfer of potentially lethal antibiotic-resistant bacteria from a food animal to a human consumer is of particular concern.

Current methods for controlling the development and spread of antibiotic-resistant bacteria include changes in antibiotic usage and patterns of usage of different antibiotics, increased government surveillance and regulation, and continued development of new and improved antibiotics. However, the ability of most bacteria to adapt to antibiotic usage and to acquire resistance to existing and new antibiotics often overcomes such conventional measures and requires the continued development of alternative means for control of antibiotic resistance in bacteria.

Antimicrobial peptides (AMPs) are a diverse group of natural compounds present in animals, plants, insects, and microorganisms. These peptides are responsible for a defense against (other) microorganisms and, as such, they may be further utilized as an alternative to the chemical preservatives. However, their use in the treatment of plant, human, and/or animal microbial infestations is up to date limited for poor availability in nature and high cost of production, low stability, and complex formulation into the suitable therapeutic product. Thus, the use of antimicrobial peptides in crop protection as well as for therapeutic applications in human and veterinary medicine is still limited.

Antimicrobial proteins exhibit a variety of three-dimensional structures, which will determine in large part their activity and stability. The stability of a certain protein is typically a critical parameter for clinical and/or industrial applications.

The initial interest in plant-derived molecules which are AMPs was followed by isolation of purothionin, the first plant-derived AMP. Caleya et al., in Appl. Microbiol, 1972, 23(5) 998-100, reported purothionin, the low molecular weight protein from wheat and barley flour, as an AMP effective against a number of phytopathogenic bacteria such as *Pseudomonas solanacearum, Xanthomonas phaseoli,* and *X. campestris, Erwinia amylovora,* and five *Corynebacterium strains.* Since then, several major groups of AMPs including thionins (types I-V), defensins, cyclotides, 2S albumin-like proteins, and lipid transfer proteins were discovered.

Plant vicilins are a commonly occurring class of plant seed storage protein. It has been shown that some vicilins are processed to produce plant defense peptides. The best characterized antimicrobial peptides produced from a vicilin are those of *Macadamia integrifolia.* Macadamia nut kernels contain a 666 amino acid (aa) vicilin protein that includes a 212-aa highly hydrophilic region proximal to an N-terminal signal sequence.

WO1998027805 discloses a family of vicilin type peptides with antimicrobial properties. Prototype proteins are of a natural origin and isolated from *Macadamia integrifolia,* as well as from some other species, including *Theobroma cacao.* In particular, two sequences marked as the 47-aa TcAMP1 *(Theobroma cacao* antimicrobial protein 1) and the 60-aa TcAMP2 *(Theobroma cacao* antimicrobial protein 2) were derived from a cocoa vicilin seed storage protein gene sequence which encodes 566 aa, and recombinantly expressed in *Escherichia coli.* However, the isolated recombinant peptides and the compositions prepared thereof had effective antimicrobial properties at high doses (5-20 µg/ml).

Marcus et al., Plant. Mol. Biol Rep (2008) 26, 75-87 investigated three *Macadamia integrifolia* and two *Theobroma cacao* peptide sequences identified in the N-proximal hydrophilic region of vicilin seed protein. The peptide's antimicrobial activity was predicted based on the presence of the characteristic C-X-X-X-C-(10-12) X-C-X-X-X-C motifs in the hydrophilic regions proximal to the N terminus. His-tagged versions of the putative peptides were expressed in *Escherichia coli.* The obtained recombinant peptides were shown to have *in vitro* antimicrobial activity against six plant pathogen strains but the effect does was very high, 9.2 - 64 µg/ml and even higher for some pathogens.

Ecuador is the most important producer of fine flavored cocoa, accounting for roughly 50 % of the world production. The fine flavored cocoa type produced in Ecuador is predominantly belonging to the Nacional variety (sometimes also called National or Arriba). Besides the intensively aromatic and traditional Nacional cocoa, a cocoa clone, CCN-51 has been cultivated in Ecuador since the 1960s. Unlike Nacional, CCN-51 is considered a bulk cocoa type because of its weaker aroma. However, it has a higher tolerance to changing climatic conditions, is resistant to different pathogens, and gives higher yields than other cocoa types. In consequence, the CCN-51 hybrid is very popular for cultivation by Ecuadorian farmers. US 2004/0172683 describes several polypeptides from the seed of cocoa bean responsible for the cocoa flavor. The present invention describes thus an unknown peptide derived from cocoa that is highly effective and active against a large spectrum of animal and plant pathogenic microorganisms. The peptide is safe to use, preferably without containing aggressive chemicals, which can be toxic and environmentally harmful.

### SUMMARY OF THE INVENTION

The present invention and embodiments thereof serve to provide a solution to one or more of the above-mentioned disadvantages. To this end, the present invention relates to a peptide having antimicrobial activity according to claim 1. More specifically, the present invention provides a peptide having a sequence according to SEQ ID N° 1. It has been surprisingly found that this peptide, obtainable from *Theobroma cacao* or being recombinantly or synthetically produced has antibacterial and/or antifungal activity against a broad spectrum of pathogens. Preferred embodiments of the peptide are shown in any of the claims 2 to 8.

In a second aspect, the present invention relates to a composition comprising a peptide according to claim 9. Preferred embodiments of the composition are shown in claim 10.

In a third aspect, the present invention relates to the use of the peptide for therapeutic purposes according to claim 11. Preferred embodiments of the use are shown in any of the claims 12 to 16. More particular, the peptide is used for infections with Gram-positive bacteria, Gram-negative bacteria, and/or fungi, preferably hyphal fungi or yeasts in humans, animals, or plants.

In a fourth aspect, the present invention relates to a method of obtaining said peptide, according to claim 17.

In a fifth aspect, the present invention relates to a vector comprising the sequence of the peptide, and that allows the expression of said peptide, according to claim 18, to a transgenic plant according to claim 19 and a method of obtaining the transgenic plant according to claim 20.

In a final aspect, the present invention relates to a seed coated with said peptide, according to claim 21.

### DESCRIPTION OF FIGURES

**Figure 1** shows a representative scheme for the detection and sequence location of peptide mass fingerprinting fragments as determined by MALDI-ToF analysis. Underlined amino acid sequences were identified as tryptic digestion products of a recombinantly expressed peptide according to SEQ ID N° 1 obtained from an SDS polyacrylamide gel.
**Figure 2** shows the LC-MS results of the sample containing the peptide according to SEQ ID N° 1. The peptide detected had a mass of 8.3 kDa in both the non-reduced (FIG 2A) and reduced (FIG 2B) samples.

### DETAILED DESCRIPTION OF THE INVENTION

The present invention concerns a peptide or composition comprising said peptide as an active ingredient for medical or pharmaceutical use, wherein said peptide has an amino acid sequence according to SEQ ID N° 1. Furthermore, the present invention relates to the use of said peptide, to a method of obtaining said peptide, and to a vector that allows the expression of said peptide.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

The term "peptide," as used herein, refers to any compound containing two or more amino acid residues joined by an amide bond formed from the carboxyl group of one amino acid residue and the amino group of the adjacent amino acid residue. The amino acid residues may have the L-form as well as the D-form, and may be naturally occurring or synthetic, linear as well as cyclic.

The term "therapeutically effective amount" is an amount that is effective to ameliorate a symptom of a disease. A therapeutically effective amount can be a "prophylactically effective amount" as prophylaxis can be considered therapy.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention and form different embodiments, as would be understood by those in the art. For example, in the following claims, any of the claimed embodiments can be used in any combination.

In a first aspect, the present invention relates to a peptide, wherein said peptide has an amino acid sequence according to SEQ ID N° 1.

In another embodiment, also peptides having an amino acid sequence exhibiting at least 95%, 96%, 97%, 98%, 99%, 99.5% sequence identity to SEQ ID N° 1 and still having the antimicrobial activity as observed for the peptide having a sequence according to SEQ ID N°1 are disclosed herein.

The term "sequence identity" as used herein refers to the extent that sequences are identical on an amino acid-by-amino acid basis over a window of comparison. Thus, a "percentage of sequence identity" is calculated by comparing two optimally aligned sequences over the window of comparison, determining the number of positions at which the identical amino acid occurs in both sequences to yield the number of matched positions, dividing the number of matched positions by the total number of positions in the window of comparison (i.e., the window size), and multiplying the result by 100 to yield the percentage of sequence identity. A gap, i.e., a position in an alignment where a residue is present in one sequence but not in the other is regarded as a position with non-identical residues. Determining the percentage of sequence identity can be done manually, or by making use of computer programs that are available in the art. An example of a useful algorithms is PILEUP. Software for performing BLAST analyses is publicly available through the National Center for Biotechnology Information (http://www.ncbi.nlm.nih.gov/).

In another embodiment, said peptide has a sequence that differs with maximally 4, more preferably 3, even more preferably 2, even more preferably 1 amino acid residues from the sequence SEQ ID N° 1, while still exhibiting the antimicrobial activity as disclosed herein.

Amino acid sequence variants of a peptide contemplated herein may be substitutional, insertional, or deletion variants. Deletion variants lack one or more residues of the peptide which may not be critical for function. Substitutional variants typically contain an alternative amino acid at one or more sites within the peptide and may be designed to modulate one or more properties of the polypeptide such as stability against proteolytic cleavage. Substitutions preferably are conservative, that is, one amino acid is replaced with one of similar size and side-chain or functional group. Conservative substitutions are well known in the art and include, for example, the changes of alanine to glycine, valine, or leucine; arginine to lysine; asparagine to glutamine; aspartate to glutamate; cysteine to methionine; glutamine to asparagine; glutamate to aspartate; glycine to proline; histidine to glutamine, tyrosine, arginine, lysine, asparagine or cysteine; isoleucine to leucine or valine; leucine to valine or isoleucine; lysine to arginine; phenylalanine to tyrosine, leucine or methionine; serine to threonine; threonine to serine; tryptophan to phenylalanine; tyrosine to tryptophan or phenylalanine; and valine to isoleucine or leucine.

By preference, the peptide of the invention is derived from or isolated from *Theobroma cacao.* More in particular, said peptide of the invention is derived from the N-terminal region of the vicilin protein of *Theobroma cacao.* Vicilin is a storage protein present and characterized in several plant species such as, but not limited to *Ananas comosus, Arachis hypogaea, Beta vulgaris, Capsicum annuum, Capsicum chinense, Carya illinoinensis, Chenopodium quinoa, Corchorus olitorius, Cucurbita maxima, Fragaria vesca, Glycine max, Gossipum arboreum, Herrania umbratica, Hordeum vulgare, Jatropha curcas, Juglans regia, Macadamia integrifolia, Macleaya cordata, Musa acuminata, Papaver somniferum, Ricinus communis, Solanum lycopersicum, Spinacia oleracea, Stenocarpus sinuatus, Theobroma cacao* and *Zea mays.* Such proteins are typically containing an extremely hydrophilic N-proximal region, and a C-terminal region. Additionally, they are produced carrying a hydrophobic N-terminal signal sequence, which is usually removed during protein maturation. The N-proximal region of said precursor protein is particularly interesting as it contains at least two, preferably four pairs of cysteine motifs (CXXXC) with the same spacing pattern.

The peptide according to the current invention was identified in experiments with recombinant peptides derived from vicilin from *Threoboma cocoa.* Without wishing to be bound to theories, said peptide is presumed to be a degradation product of one of those recombinant peptides.

While the peptide as disclosed herein may be obtained by means of recombinant expression, it may also be purified from *Theobroma cacao* varieties, more preferably variety CCN-51. Surprisingly, the cacao variety CCN-51 or "lavados", known as highly resistant towards microbial infestations, was shown to be particularly rich in vicilin-derived N-terminal peptide.

In another or further embodiment, said peptide comprises a signal peptide at its N terminus. Preferably, said signal peptide has a sequence according to SEQ ID N° 2.

In another embodiment, said signal peptide exhibits at least 95%, 96%, 97%, 98%, 99% or more preferably 100% sequence identity to SEQ ID N° 2. Alternatively, said peptide has a signal peptide sequence that differs with maximally 3, more preferably maximally 2, even more preferably 1 amino acid residues from the sequence SEQ ID N° 2.

In an embodiment, the peptide of the invention containing a signal peptide has a sequence according to SEQ ID N° 3.

In another embodiment, the peptide is a recombinant or a synthetic peptide. A suitable expression system, such as *Escherichia coli,* or another suitable system known in the art, upon insertion of a vector containing a coding sequence for a peptide with an SEQ ID N° 1, gives rise to a peptide. Any suitable protein/peptide synthesis method known in the art can be employed to synthesize the peptide of the invention.

As disclosed but not claimed, the peptide as described herein is provided with an affinity tag at its N and/or C terminus. Affinity tags are used for affinity purification of recombinant proteins and peptides expressed in *Escherichia coli* and other systems. Said tag can be a poly-histidine-tag, which is an amino acid motif that consists of at least six histidine (His) residues.

Alternatively and without being limitative, the peptide can be tagged with an HQ tag that alternates histidine and glutamine residues(HQHQHQ), an HN tag that alternates histidine and asparagine (HNHNHNHNHNHN), a HAT tag (KDHLIHNVHKEEHAHAHNK), an ALFA tag (SRLEEELRRRLTE), an Avi tag (GLNDIFEAQKIEWHE), a C-tag (EPEA), a Calmodulin-tag (KRRWKKNFIAVSAANRFKKISSSGAL), a polyglutamate tag, a polyarginine tag, an E-tag (GAPVPYPDPLEPR), a FLAG-tag (DYKDDDDK), an HA-tag (YPYDVPDYA), a Myc-tag (EQKLISEEDL), a NE-tag (TKENPRSNQEESYDDNES), a Rho1D4 tag (TETSQVAPA), an S-tag (KETAAAKFERQHMOS), an SBP tag (DEKTTGWRGGHVVEGLAGELEQLRARLEHHPQGQREP) or a Strep-tag (WSHPQFEK). Analysis of the peptide of the invention showed that the peptide has antibacterial and/or antifungal activity.

The antimicrobial peptide per se has a particular three-dimensional structure which may be determined using X-ray crystallography or nuclear magnetic resonance techniques. Without wanting to be bound to theory, it is believed that this structure plays a role in the observed activity of said peptide. Alpha-helical plant-derived antimicrobial peptides (AMPs), to which the peptide of the invention belongs, often have amphipathic helices with one face of the helix predominantly hydrophilic and the other face predominantly hydrophobic. This structure is regularly found in AMPs that disrupt cell membranes to cause leakage of the cell content and lysis and in AMPs that enter the cells to attack other structures within the cell. Alpha-helical AMPs are usually structurally disordered in solution, which facilitates passage of the dense net of the cell wall. Upon binding to the cell membrane that is hydrophobic and charged below the lipid heads, secondary structures (alpha-helices) are formed which enables the protein to penetrate the cell membrane. This order/disorder transition is predominantly regulated by the length of the hydrophobic domain within the alpha-helices. Too long hydrophobic helices lead to ordered structures in solution which has a negative impact on AMP activity.

In an embodiment, said peptide is active against Gram-positive and Gram-negative bacteria and fungi, preferably hyphal fungi or yeasts. Without being limitative, the peptide is active against bacteria selected from the group comprising: *Acinetobacter* spp., *Bartonella* spp., *Bordetella* spp., *Borrelia* spp., *Brucella* spp., *Campylobacter* spp., *Chlamydia* spp., *Clostridium* spp., *Corynebacterium* spp., *Enterococcus* spp., *Enterobacter* spp., *Erwinia* spp., *Escherichia* spp., *Francisella* spp., *Haemophilus* spp., *Helicobacter* spp., *Klebsiella* spp., *Legionella* spp., *Leptospira* spp., *Listeria* spp., *Mycobacterium* spp., *Mycoplasma* spp., *Neisseria* spp., *Rickettsia* spp., *Salmonella* spp., *Shigella* spp., *Staphylococcus* spp., *Streptococcus* spp., *Treponema* spp., *Ureaplasma* spp., *Vibrio* spp., *Yersinia* spp., *Acidovorax* spp., *Agrobacterium* spp., *Arthrobacter* spp., *Bacillus* spp., *Burkholderia* spp., *Clavibacter* spp., *Cronobacter* spp., *Curtobacterium* spp., *Lefisonia* spp., *Pantoea* spp., *Paenibacillus* spp., *Pectobacterium* spp., *Phytoplasma* spp., *Proteus* spp., *Pseudomonas* spp., *Ralstonia* spp., *Rhizobacter* spp., *Rhizomonas* spp., *Rhodococcus* spp., *Serratia* spp., *Sphingomonas* spp., *Spiroplasma* spp., *Streptomyces* spp., *Xanthomonas* spp., *Xylella* spp., or *Xylophilus* spp.

Without wishing to be limitative, the peptide of the invention is active against fungi selected from the group comprising: *Ajellomyces* spp., *Aspergillus* spp., *Basidiobolus* spp., Blastomyces spp., *Candida* spp., *Coccidioides* spp., *Conidiobolus* spp., *Cryptococcus* spp., *Emmonsia* spp., *Histoplasma* spp., *Hanseniaspora* spp., *Lacazia* spp., *Paracoccidioides* spp., *Pneumocystis* spp., *Sporothrix* spp., *Stachybotrys* spp., *Talaromyces* spp., *Acrocalymma* spp., *Aecidium* spp., *Albonectria* spp., *Allodus* spp., *Alternaria* spp., *Amphobotrys* spp., *Apiosporina* spp., *Armillaria* spp., *Blumeria* spp., *Botryotinia* spp., *Botrytis* spp., *Ceratosystis* spp., *Colletotrichum* spp., *Cryptosporiopsis* spp., *Exobasidium* spp., *Fusarium* spp., *Hypocrea* spp., *Leptosphaeria* spp., *Magnaporthe* spp., *Melampsora* spp., *Meyerozyma* spp., *Monilinia* spp., *Mycospharella* spp., *Microsphaera* spp., *Mucor* ssp., *Penicillium* spp., *Pichia* spp., *Phytophtora* spp., Saccharomyces spp., *Sporobolomyces* spp., *Plasmodiophora* spp., *Podosphaera* spp., *Puccinia* spp., *Pythium* spp., *Rhizoctonia* spp., *Sclerotinia* spp., *Septoria* spp., *Taphrina* spp., *Thanatephorus* spp., *Torulaspora* spp., *Uromyces* spp., *Ustilago* spp., *Venturia* spp., or *Verticillium* spp.

In a second aspect, the present invention provides compositions comprising a peptide as described in the paragraphs above. Said compositions are particularly suitable for pharmaceutical and veterinary applications, and/or for crop protection.

In an embodiment, the compositions are liquid, semisolid, solid, or gaseous and/or is in a dosage form of a tablet, capsule, powder, granulate, aerosol, paste, syrup, suspension, emulsion, or solution. Non-limiting examples of said compositions are soluble powders, soluble granules, wettable granules, tablet formulations, dry flowables, aqueous flowables, wettable dispersible granules, oil dispersions, suspension concentrates, dispersible concentrates, emulsifiable concentrates, aqueous suspensions, fertilizer granule, or sprayable compositions. Said compositions may be formulated such that it is suited for oral, injectable, intravenous, intramuscular, cutaneous, inhalation, topical or intranasal administration. In other embodiment, said compositions are formulated to allow coating of one or more objects, spraying, spray coating, evaporating, nebulising, atomising, suspending, diluting, etc.

In some embodiments, the compositions comprise a pharmaceutically acceptable carrier, excipient, or diluent. By preference, said excipients are chosen from fillers, binders, disintegrants, sweeteners, coatings, lubricants, and/or glidants. Diluents or fillers increase the bulk of solid compositions and may make a dosage form containing the compositions easier for the patient and caregiver to handle. Diluents suitable for tablets according to the current invention include, for example, microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol, and talc.

Solid compositions that are compacted into a dosage form, such as a tablet, may include excipients whose functions include helping to contain the active ingredient and other excipients together after compression. Suitable binders include acacia, alginic acid, carbomer (e.g. carbopol), carboxymethylcellulose sodium, dextrin, ethylcellulose, gelatin, guar gum, hydrogenated vegetable oil, hydroxyethyl cellulose, hydroxypropyl cellulose (e.g. Klucel^{®}), hydroxypropyl methylcellulose (e.g. Methocel^{®}), liquid glucose, magnesium aluminum silicate, maltodextrin, methylcellulose, polymethacrylates, povidone (e.g. Kollidon^{®}, Plasdone^{®}, pregelatinized starch, sodium alginate, and starch.

The dissolution rate of compacted solid compositions may be increased by the addition of a disintegrant to the compositions. Suitable disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Glidants can be added to improve the flowability of non-compacted solid compositions and to improve the accuracy of dosing. Excipients that may function as glidants include colloidal silicon dioxide, magnesium trisilicate, magnesium stearate, powdered cellulose, starch, talc, and tribasic calcium phosphate.

When a dosage form such as a tablet is made by the compaction of a powdered composition, the composition is subjected to pressure from a punch and dye. Some excipients and active ingredients tend to adhere to the surfaces of the punch and dye, which can cause the product to have pitting and other surface irregularities. A lubricant can be added to the compositions to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate. Preferably, said lubricant is present in between 0.25 and 1% (w/w) by weight.

Flavoring agents and flavor enhancers make the dosage form more palatable to the patient. Common flavoring agents and flavor enhancers for pharmaceutical products that may be included in the compositions as disclosed herein include maltol, vanillin, ethyl vanillin, menthol, citric acid, fumaric acid, ethyl maltol and tartaric acid.

Solid compositions may also be dyed using any pharmaceutically acceptable colorant to improve their appearance and/or facilitate, patient identification of the product and unit dosage level.

In an embodiment and specifically when the compositions are intended for agricultural use, said excipients are agricultural compatible excipients. Said "agricultural compatible carriers" or "agricultural compatible excipients" which can be regarded as a vehicle, is generally inert and it must be acceptable in agriculture. Thus, the phrase "agriculturally compatible" denotes a substance that can be used routinely under field conditions without interfering with growers' planting equipment, and without adversely influencing crop development or the desired ecological balance in a cultivated area.

The agriculturally compatible carriers or excipients can be solid. Solid carriers or excipients can include but are not limited to clays, natural or synthetic silicates, silica, resins, waxes, solid fertilizers, a polymer, a granular mass, perlite, a perlite granule, peat, a peat pellet, soil, vermiculite, charcoal, sugar factory carbonation press mud, rice husk, carboxymethyl cellulose, fine sand, calcium carbonate, flour, alum, a starch, talc, polyvinyl pyrrolidone, or a combination thereof. The agriculturally compatible carrier or excipient can be a liquid. Liquid carriers or excipients can include but are not limited to water, alcohols, ketones, petroleum fractions, oils, aromatic or paraffinic hydrocarbons, chlorinated hydrocarbons, liquefied gases or a combination thereof. More particularly, the agriculturally compatible carriers or excipients can include a dispersant, a surfactant, an additive, a thickener, an anti-caking agent, residue breakdown, a composting formulation, a granular application, diatomaceous earth, a coloring agent, a stabilizer, a preservative, a polymer, a coating or a combination thereof. A person skilled in the art can readily determine the appropriate carrier or excipient to be used taking into consideration factors such as a particular compound, plant to which the inoculum is to be applied, type of soil, climate conditions, whether the inoculum is in liquid, solid or powder form, and the like. The additive can comprise an oil, a gum, a resin, a clay, a polyoxyethylene glycol, a terpene, a viscid organic, a fatty acid ester, a sulfated alcohol, an alkyl sulfonate, a petroleum sulfonate, an alcohol sulfate, a sodium alkyl butane diamate, a polyester of sodium thiobutant dioate, a benzene acetonitrile derivative, a proteinaceous material, or a combination thereof. The proteinaceous material can include a milk product, wheat flour, soybean meal, blood, albumin, gelatin, or a combination thereof. The thickener can comprise a long chain alkylsulfonate of polyethylene glycol, polyoxyethylene oleate or a combination thereof. The surfactant can contain a heavy petroleum oil, a heavy petroleum distillate, a polyol fatty acid ester, a polyethoxylated fatty acid ester, an aryl alkyl polyoxyethylene glycol, an alkyl amine acetate, an alkyl aryl sulfonate, a polyhydric alcolhol, an alkyl phosphate, or a combination thereof. The anti-caking agent can include a sodium salt such as a sodium sulfite, a sodium sulfate, a sodium salt of monomethyl naphthalene sulfonate, or a combination thereof; or a calcium salt such as calcium carbonate, diatomaceous earth, or a combination thereof.

In an embodiment, said compositions further comprises one or more of the following: water, other nutritive substance, weak acid, vegetable oil, essential oil, metabolism stimulant, emulsifying agent, viscosity agent, tinting material, suspending agent, dispersion agent, preservative, complexing agent, stabilizer, carrier, vehicle or wetting agent, or any combination thereof. In an embodiment, said compositions further comprises at least one oil, surfactant and polymer.

The compositions may be manufactured in a conventional manner. In certain embodiments, the compositions provided herein includes one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide, and cetylpyridinium chloride.

The peptides as described herein can be formulated in the compositions at an amount of between 0.1 and 50 µg/ml, preferably between 0.1 and 45 µg/ml, preferably between 0.1 and 40 µg/ml, preferably between 0.1 and 35 µg/ml, preferably between 0.1 and 30 µg/ml, preferably between 0.1 and 25 µg/ml, preferably between 0.1 and 20 µg/ml, preferably between 0.1 and 15 µg/ml, preferably between 0.1 and 10 µg/ml, preferably between 0.1 and 9 µg/ml, preferably between 0.1 and 8 µg/ml, preferably between 0.1 and 7 µg/ml, preferably between 0.1 and 6 µg/ml, preferably between 0.1 and 5 µg/ml, preferably between 0.1 and 4 µg/ml, preferably between 0.1 and 3 µg/ml, preferably between 0.1 and 2 µg/ml, preferably between 0.1 and 1 µg/ml, preferably between 0.1 and 0.9 µg/ml, preferably between 0.1 and 0.8 µg/ml, preferably between 0.1 and 0.7 µg/ml, preferably between 0.1 and 0.6 µg/ml, preferably between 0.1 and 0.5 µg/ml or preferably between 0.1 and 0.4 µg/ml.

Alternatively, the peptides can be formulated in the compositions at an amount of between 0.2 and 50 µg/ml, preferably between 0.3 and 50 µg/ml, preferably between 0.4 and 50 µg/ml, preferably between 0.5 and 50 µg/ml, preferably between 0.6 and 50 µg/ml, preferably between 0.7 and 50 µg/ml, preferably between 0.8 and 50 µg/ml, preferably between 0.9 and 50 µg/ml, preferably between 1 and 50 µg/ml, preferably between 2 and 50 µg/ml, preferably between 3 and 50 µg/ml, preferably between 4 and 50 µg/ml, preferably between 5 and 50 µg/ml, preferably between 6 and 50 µg/ml, preferably between 7 and 50 µg/ml, preferably between 8 and 50 µg/ml, preferably between 9 and 50 µg/ml, preferably between 10 and 50 µg/ml, preferably between 15 and 50 µg/ml, preferably between 0.5 and 20 µg/ml, preferably between 25 and 50 µg/ml, preferably between 30 and 50 µg/ml, preferably between 35 and 50 µg/ml, preferably between 40 and 50 µg/ml, preferably between 45 and 50 µg/ml,

In another embodiment, the compositions contain at least 0.1% by weight of the peptide, more preferably at least 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 99.5% by weight of peptide.

In a third aspect, the peptides or compositions as described herein are suitable for therapeutic use. "Therapeutic use" as described herein refers to the use of the peptides or the compositions thereof for ameliorating the symptoms of a disease in a human or non-human animal. In practicing the methods of treatment or use provided herein, a therapeutically effective amount of pharmaceutical composition described herein is administered to a subject such as a mammal or non-mammal having a disease, disorder, or condition to be treated. In some embodiments, the subject is a human. A therapeutically effective amount may vary widely depending on the severity of the disease, the age and relative health of the subject, the potency of the therapeutic agent used, and other factors. The therapeutic agents, and in some cases, compositions described herein, may be used singly or in combination with one or more therapeutic agents as components of mixtures.

In an embodiment, the peptides as described herein are used in pharmaceutical compositions for treatment or prevention of infections caused by a broad range of microorganisms including Gram-positive, Gram-negative bacteria, and/or fungi, preferably hyphal fungi or yeasts in a subject in need thereof. Said subject may be a human or animal. Said pharmaceutical compositions may contain a therapeutically effective amount of the antimicrobial peptide and a suitable carrier.

The peptides or compositions described herein may be administered to a subject by appropriate administration routes, including but not limited to, intravenous, intraarterial, oral, parenteral, buccal, topical, transdermal, rectal, intramuscular, subcutaneous, intraosseous, transmucosal, inhalation, or intraperitoneal administration routes. The composition described herein may include, but are not limited to, aqueous liquid dispersions, self-emulsifying dispersions, solid solutions, liposomal dispersions, aerosols, solid dosage forms, powders, immediate-release formulations, controlled release formulations, fast melt formulations, tablets, capsules, pills, delayed-release formulations, extended-release formulations, pulsatile release formulations, multi-particulate formulations, and mixed immediate and controlled release formulations.

In an embodiment, the peptides or compositions as described herein may be added to animal feed to reduce potential microbial infections in livestock. In another embodiment, the peptides or compositions described herein are formulated for oral, topical, or parenteral administration to treat microbial infections in veterinary medicine.

In another embodiment, the peptides or compositions as described herein are suitable for use in crop protection, as a feed or food additive, for pharmaceutical use, as a preservative, as a decontaminant and/or in cosmetical applications.

When used for crop protection, the peptides or the compositions as described herein are formulated with an agricultural compatible carrier or agricultural compatible excipient according to the embodiments above described. The person skilled in the art will determine the right dose, form, and method of application of said composition, in the function of the treated crop and the pathogen targeted.

In an embodiment, the peptides or compositions described herein are incorporated in feed or food additives to prevent pathogen spreading via feed and food. It is known that feed may carry pathogens that are detrimental to animal health and welfare. Antiseptic products such as formaldehyde are commonly used to disinfect the feed but these products can, in turn, exert negative effects. The antimicrobial peptides as disclosed herein are a safe alternative.

In another embodiment, the peptides or the compositions as described herein are suitable to be used as a decontaminant or disinfectant of surfaces, tools, instruments, devices, objects, or body parts. In an embodiment, such devices or objects include, but are not limited to, linens, cloth, plastics, latex fabrics, natural rubbers, implantable devices, surfaces, or storage containers. In a particular embodiment, the peptides or the compositions as disclosed herein are incorporated in cleaning agents, detergents, soaps, or sprays.

In certain embodiments, the peptides as described herein can be incorporated into various health care products as well. For example, the peptides can be incorporated into toothpaste, mouthwash, shampoo, soap, cream, or antiperspirant to reduce or prevent microbial colonization or re-establishment of the oral cavity or skin.

In another embodiment, the peptides and compositions as described herein may be used as a food, feed, cosmetics, or pharmaceutical preservative or in treating food products to control, reduce, or eliminate potential pathogens or contaminants.

In another embodiment, the peptides or compositions as described herein are used for controlling a pathogen in a plant, preferably in a crop wherein said pathogens are Gram-positive and Gram-negative bacteria and/or fungi, preferably hyphal fungi or yeasts. Said plant can be a monocotyledonous or a dicotyledonous plant, including fodder or forage legumes, ornamental plants, food crops, trees or shrubs. In an embodiment, said plant is preferably a crop. Preferably, said plant belongs to the group of *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis* spp., *Artocarpus* spp., *Asparagus officinalis, Avena* spp., *Averrhoa carambola, Bambusa* spp., *Benincasa hispida, Bertholletia excelsea, Beta vulgaris, Brassica* spp., *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., Corchorus spp., *Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis* spp., *Eleusine coracana, Eragrostis tef, Erianthus* spp., *Eriobotrya japonica, Eucalyptus* spp., *Eugenia uniflora, Fagopyrum* spp., *Fagus* spp., *Festuca arundinacea, Ficus carica, Fortunella* spp., *Fragaria* spp., *Ginkgo biloba, Glycine* spp., *Gossypium hirsutum, Helianthus* spp., *Hemerocallis fulva, Hibiscus* spp., *Hordeum* spp., *Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp., *Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp., *Panicum miliaceum, Panicum virgatum, Passiflora edulis, Pastinaca sativa, Pennisetum* spp., *Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix* spp., *Sambucus* spp., *Secale cereale, Sesamum* spp., *Sinapis* spp., *Solanum* spp., *Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp., *Tropaeolum minus, Tropaeolum majus, Vaccinium* spp., *Vicia* spp., *Vigna* spp., *Viola odorata, Vitis* spp., *Zea mays, Zizania palustris, Ziziphus* spp.; including the progenies and hybrids between the above.

The peptides or compositions as described herein are suitable to be introduced to a plant, a plant part or a substrate comprising or hosting said plant, thereby conferring pathogen resistance or pathogen control to said plant. Said introduction may be artificial.

Methods for introducing the peptides or the compositions thereof to plants may include: treating the plant and/or a plant part and/or growth medium wherein said plant is grown, inoculation of the seeds, coating the seeds, direct inoculation of plants or plant parts, spraying or wetting plants or plant parts (eg. ears). An appropriate method may be chosen depending on the type of plant to which the peptide or composition is to be introduced.

As another of further non-limiting example, the peptides or compositions as described herein may be applied in the form of coatings. The coating may be applied to the seed by spraying on the seeds or soaking said seeds in a solution containing the peptide or composition. In another example, for coating seeds with a solution as described in the previous example, a binding agent may be added, such as a binding agent comprising carbide (calcium carbonate).

In embodiments, the coating may be applied to a naked and untreated plant part. In other embodiments, the coating may be applied as an overcoat to a previously treated plant part. Seed coatings are particularly preferred in the treatment of soil-borne fungal diseases. In embodiments, the seed coating may be applied to a naked and untreated seed. In other embodiments, the seed coating may be applied as a seed overcoat to a previously treated seed.

In an embodiment, the peptides or compositions as described herein may be applied to the soil or any other substrate in which said plant grows in order to remove pests and/or pathogens from said substrate.

Inoculating the substrate comprising or hosting said plant or plant part can be performed, by way of example and without the intention to be limiting, using a powder, a granule, a pellet, a plug, or a soil drench that is applied to the substrate. Inoculation could also be performed by a liquid application, such as a foliar spray or liquid composition. The application may be applied to a growing plant or to the substrate. Plants, in particular agricultural plants, can be grown in the substrate. In one embodiment, said substrate is soil, sand, gravel, polysaccharide, mulch, compost, peat moss, straw, logs, clay, or a combination thereof. In another embodiment, the substrate can also include a hydroculture system or an *in vitro* culture system. In some embodiments, a combination of different application methods as described herein is applied.

In an embodiment, the peptides or the compositions as described herein are suitable for use in treating or preventing pathogenic infections wherein the pathogens are selected from the group consisting of Gram-positive bacteria, Gram-negative bacteria, and/or fungi as described in any of the previous embodiments.

The invention as disclosed herein also relates to a plant seed coated with the peptide or composition described in any of the previous embodiments.

In another aspect, the invention relates to methods for obtaining the peptides as disclosed herein, wherein said peptides are recombinant or synthetic peptides, or are derivable (e.g by extraction or precipitation) from *Theobroma cacao, more preferably* from the variety CCN-551. These methods are known in the art.

In an embodiment, the peptide of interest may be dried by lyophilization.

In an embodiment, the peptides are concentrated. Preferably, said peptides are concentrated by standard salt or organic solvent precipitation. Alternatively, the peptides are concentrated by dialysis against a volatile buffer (e.g., ammonium carbonate), by using a filter-based concentrator, or by ion-exchange chromatography.

The quantification of the peptide content is done by spectrophotometric methods such as the Lowry method or Bradford assay, or any other method known in the art. In another embodiment, the quantification of the peptide is done by any suitable chromatographic method known in the art. In a preferred embodiment, an LC-MS method is used for the quantification of the peptide content. In a preferred embodiment, MALDI-TOF analyses are conducted on fragments generated via tryptic digestion of the proteins obtained from cacao extracts. In one embodiment, the data obtained from MALDI-MS tryptic digest analyses is further corroborated by preparing and performing immunochemical assays to detect specific differences between samples from.

In a further preferred embodiment, said peptide is substantially free of other peptides. The term "substantially free", as used herein, means at least 95 wt.% free of other peptides; preferably at least 99 wt.% free of other peptides. In a preferred embodiment, said peptide is present in a crystal and/or a solid form.

In anotheraspect, the present invention relates to a vector comprising a coding sequence for a peptide having a sequence according to SEQ ID N° 1, and optionally comprising a sequence for an N-terminal signal peptide, said signal peptide sequence having a sequence according to SEQ ID N° 2 wherein said vector is designed to allow expression of said peptide in an expression system. In another preferred embodiment said vector comprises a purification tag as disclosed in a previous embodiment.

In an embodiment, the DNA extracted from cacao beans is used to amplify the coding sequence of the peptide, which is cloned in a suitable expression vector such as any plasmid or virus designed for gene expression in cells.

The peptides as described herein can thus be expressed in any suitable system for the purpose of producing the peptide for further use. Suitable hosts for the expression of the protein include *Escherichia coli,* fungal cells, insect cells, mammalian cells, and plants. Standard methods for expressing proteins in such hosts are described in a variety of texts including section 16 (Protein Expression) of *Current Protocols in Molecular Biology* (supra).

In general, DNA encoding the amino acid sequence of interest is contained in an expression vector, in some cases linked in-frame at the 5' or 3' end to another coding sequence so as to encode a peptide having a sequence according to SEQ ID N° 1, and optionally comprising at the N terminus a SEQ ID N° 2. The total coding sequence is operably linked to a promoter such that the promoter drives the expression of the coding sequence. The coding sequence is also referred to herein as the "target gene".

According to an embodiment, the promoter is either a promoter native to the microorganism (for example the *Escherichia coli* trpE promoter), a synthetic promoter such as the Tac promoter, or a promoter obtainable from a heterologous organism, for example, a virus, a bacterium or a bacteriophage such as a phage λ or T7 which is capable of functioning in the microorganism. The promoter may be constitutive or, more preferably, inducible. The expression vector may also contain a selectable marker gene, which may be an antibiotic resistance gene such as an ampicillin, tetracycline, chloramphenicol, or kanamycin resistance gene.

Many promoter systems are available, often from commercial sources, which are suitable for the expression of peptides of the invention in *Escherichia coli.* For example, the P_{BAD} promoter from the *araBAD* (arabinose) operon has advantageous induction properties, being inducible 1,200-fold over background (Guzman et al., 1995). Others include P_{LAC}, P_{TAC}, P_{TRC}, PL and PR The P_{TAC} promoter is a hybrid derived from the trp and lac promoters of *Escherichia coli,* and is one of the most potent *Escherichia* coli-based promoter systems known. It is inducible by IPTG, as with the lac promoter.

The preferred host cells of the present invention are of *Escherichia coli* M15 host strain. In another preferred embodiment, Pichia pastoris are used as host cells for the expression of the peptides of the invention.

In a further preferred embodiment, the selection of clones is done and overexpressed peptides are extracted via their affinity tags, using Ni-NTA agarose. The recombinant peptides are separated and purified using the same methods applied for the peptides isolated from natural sources in a previous embodiment.

Expression of the new peptide in a genetically engineered cell will typically result in a product having a three-dimensional structure identical to naturally occurring peptide, which is isolated from cacao plant material. This three-dimensional structure includes correctly formed intramolecular disulfide linkages between cysteine residues. However, even if the protein is chemically synthesized, methods are known in the art for further processing of the protein to break undesirable disulfide bridges and form the bridges between the desired cysteine residues to give the desired three-dimensional structure should this be necessary.

In another embodiment of the invention, said peptide can be obtained by chemical synthesis using the known peptide-synthetizing techniques, such as solid-phase syntheses on instruments such as room-temperature peptide synthesizers, microwave peptide synthesizers, parallel peptide synthesizers and the like.

In one embodiment of the invention, it is possible to express the peptide as described herein in transgenic plants. Consequently, the current invention also relates to transgenic plants expressing one or more peptides as described herein.

In an embodiment, the transgenic plant expresses a peptide exhibiting at least 95% sequence identity to SEQ ID N° 1 or that differs with maximally 3 amino acids from the sequence according to SEQ ID N° 1. In a further embodiment, said peptide comprises a sequence for an N terminal signal peptide, said signal peptide sequence having a sequence identity of 95% to SEQ ID N° 2.

In a further embodiment, the transgenic plant expresses a peptide having 96%, more preferably 97%, more preferably 98%, more preferably 99% or more preferably 100% identity to SEQ ID N° 1. In an embodiment said peptide comprises a purification tag as disclosed in a previous embodiment.

In an embodiment said peptide comprises a purification tag as disclosed in a previous embodiment.

According to the present invention, plant cells can be transformed with DNA constructs according to a variety of known genetic engineering methods *(Agrobacterium tumefaciens* transformation, Ti plasmids, electroporation, micro-injections, micro-projectile gun, PEG-mediated transformation and the like). A genetic engineering method as used herein may be understood as any methods used to introduce foreign DNA sequences into plant cells, in order to regenerate genetically modified plants that express desired traits or characteristics.

In one embodiment of the invention, DNA sequences encoding for a peptide having a sequence according to SEQ ID N°1 or DNA coding for homologs from other plant species, can be used in conjunction with a DNA sequence encoding a preprotein from which the mature protein is produced. This preprotein contains a native peptide sequence, which will target the protein to a particular cell compartment (e.g., the apoplast or the vacuole). These coding sequences can be ligated to a plant promoter sequence that will ensure strong expression in plant cells. This promoter sequence might ensure strong constitutive expression of the protein in most or all plant cells, it may be a promoter that ensures expression in specific tissues or cells that are susceptible to microbial infection and it may also be a promoter that ensures strong induction of expression during the infection process. These types of gene cassettes will also include a transcription termination and polyadenylation sequence 3' of the antimicrobial protein-coding region to ensure efficient production and stabilization of the mRNA encoding the antimicrobial proteins. It is possible that the efficient expression of the antimicrobial peptide disclosed herein might be facilitated by the inclusion of their individual DNA sequences into a sequence encoding a much larger peptide.

Gene cassettes encoding the peptides are expressed in plant cells using methods known in the art. Firstly, the gene cassettes can be ligated into binary vectors carrying: i) left and right border sequences that flank the T-DNA of the *Agrobacterium tumefaciens* Ti plasmid; ii) a suitable selectable marker gene for the selection of antibiotic-resistant plant cells; iii) an origin of replication that functions in both *Agrobacterium tumefaciens* and *Escherichia coli;* and iv) antibiotic resistance genes that allow selection of plasmid-carrying cells of *Agrobacterium tumefaciens* and *Escherichia coli.* Without wishing to be limitative, the DNA sequence of the peptides disclosed herein can be cloned in any binary vectors known in the art, such as plasmids (pEXA128, pBR322, pUC19), bacteriophage (λ phage, M13 phage), cosmids, BACs or YACs. The binary vector carrying the DNA sequence of the peptides can be introduced by either heat shock, electroporation or triparental mating into *Agrobacterium tumefaciens* strains carrying disarmed Ti plasmids such as strains LBA4404, GV3101, and AGL1 or into *Agrobacterium rhizogenes* strains such as A4 or NCCP1885. These *Agrobacterium* strains can then be co-cultivated with suitable plant explants or intact plant tissue and the transformed plant cells and/or regenerants selected using antibiotic resistance. Alternatively, the binary vector carrying the DNA sequence of the peptides can be overexpressed in bacterial or fungal cells.

The present disclosure also relates to a method of obtaining a transgenic plant expressing a peptide exhibiting at least 95% sequence identity to SEQ ID N° 1 or that differs with maximally 3 amino acids from the sequence according to SEQ ID N° 1 and optionally comprising a sequence for an N terminal signal peptide, said signal peptide sequence having a sequence identity of 95% to SEQ ID N° 2.

The method comprises the steps of:
(a) optionally isolating or synthesizing a nucleic acid sequence encoding a peptide exhibiting at least 95% sequence identity to SEQ ID N° or that differs with maximally 3 amino acids from the sequence according to SEQ ID N° 1;
   and incorporating the nucleic acid sequence into a suitable expression vector;
(b) introducing the nucleic acid into at least a plant cell by a genetic engineering method to produce a transformed plant cell expressing said peptide;
(c) regenerating the transformed plant cell into whole transgenic plants; and
(d) selecting and identifying transgenic plants expressing said peptide using suitable screening methods.

In another embodiment of the method disclosed herein, the DNA sequence of the peptides is introduced into the plant cell by biolistic bombardment. Biolistic bombardment involves introducing foreign DNA sequences into plant cells using a gene gun. The DNA sequence encoding the antimicrobial peptide as disclosed herein is attached to metal particles, such as gold particles, and then bombarded into the plant cells.

In yet another embodiment, the DNA sequence of the peptides is introduced into the plant cell by electroporation which involves the use of electrical pulses to create pores in the cell membrane, allowing the DNA sequence encoding the antimicrobial peptide to enter said cell. Alternatively the DNA sequence encoding the antimicrobial peptide may be directly injected into the plant cell (microinjection).

In yet another alternative embodiment the DNA sequence encoding said peptide is introduced into the plant cells by PEG-mediated transformation that involves the use of polyethylene glycol (PEG) to create temporary pores in the cell membrane, allowing the DNA sequence to enter the cell.

Any plant organ, tissue or explant may be used with the genetic engineering method in order to obtain the transgenic plant as disclosed herein. Non limiting examples include: individual cells, callus, seeds, leaf explants, stem explants, root explants, embryonic tissue, meristems or pollen.

Overall, the specific genetic engineering method and explant used to obtain the transgenic plant expressing the peptide as disclosed herein depend on factors such as the plant species, the efficiency of the transformation method, and the desired level of expression of the antimicrobial peptide.

In a further embodiment of the method as disclosed herein the transformed cell is regenerated into an entire plant expressing the peptide.

The transgenic plants expressing the peptide are identified and selected. This step is essential for eliminating the non-transformed plans. Antibiotic or herbicide resistance selection is one of the most commonly used selection methods for transgenic plants. The transgenic plants are transformed with a gene that confers resistance to an antibiotic or herbicide and are then grown on a medium containing said antibiotic or herbicide, which kills or inhibits the growth of the non-transformed plants. In some embodiments, the transgenic plants are transformed with a gene that encodes the reporter protein and can then be easily identified and selected based on the expression of the reporter gene.

In other embodiments, the transgenic plants are selected by nutritional selection. This method is based on the ability of the transgenic plants to grow on media lacking a specific nutrient that is essential for the growth of non-transformed plants. The transgenic plants are transformed with a gene that confers the ability to synthesize the missing nutrient.

Marker-free selection involves the use of selectable markers that can be removed from the transgenic plants after selection. This can be achieved using site-specific recombination systems, such as the Cre-lox system, which allows for the removal of the selectable marker gene without leaving any trace of the foreign DNA.

The invention is further described by the following non-limiting examples which further illustrate the invention, and are not intended to, nor should they be interpreted to limit the scope of the invention.

### EXAMPLES

### Example 1: Peptide production

Total DNA was extracted from embryos of the CCN-51 cocoa beans and by using primers Forward_BamH1-5'CGCGGATCCTATGGCAGAAAACAATAT3' and Reverse_Kpn1-5'GGTACCTCATTGCCTTTGAAGCTCTTCTTCTT3' a 171-bp DNA fragment was amplified (DNA of interest).

The amplified DNA fragment was ligated into the BamH1/Kpn1 sites of the cloning vector pEXA128. Both the plasmid caring the DNA of the peptide and the pQE30 overexpression plasmid were transformed into *E. coli* DH5a cells for amplification. The plasmids were extracted from overnight cultures, were digested with BamHI and KpnI at 37 °C for 2 hours and were separated on 1% agarose gels. Linearized pQE30 plasmids and inserts from the pEXA128 plasmids were extracted from the gels and inserts were ligated into the pQE30 plasmid. The pQE30 overexpression plasmid encodes the HIS tag, and overexpression of the peptide of invention will give rise to a fusion peptide consisting of the peptide and an N-terminal HIS tag. The resulting plasmids were transformed into *E. coli* M15 overexpression cells. Plasmids that were extracted from overnight cultures were sequenced and only clones that matched the expected sequences were used for overexpression. 16 ml overnight cultures were used to inoculate 800 ml liquid LB medium at 37 °C. Once an OD₆₀₀ of between 0.6 and 0.8 was reached, IPTG was added to a final concentration of 1 mM to induce overexpression. The cells overexpressing the peptides were incubated for 5 hours at 37 °C. Cells were lysed and the supernatant subjected to protein extraction followed by affinity chromatography using Ni NTA agarose columns. After elution of HIS-tagged proteins using imidazole, the purified peptide was separated according to its molecular weight using SDS-PAGE. In short, 25 µl of the protein sample was mixed with 5 µl of 6X sample buffer containing bromophenol blue as tracking dye. The mixture was heated at 95°C for 5 minutes and loaded on SDS-PAGE gels (83 mm×65 mm×1mm) containing 12.5% or 15% (w/v) acrylamide. Electrophoresis was performed at 130 V for 90 min. After electrophoresis, the gel was either stained with Coomassie^{®} Blue (45% (v/v) methanol, 10% acetic acid, 2.93 x 10⁻³ M Coomassie^{®} Brilliant Blue G-250) for 20 min or blotted electrophoretically onto PVDF membranes as described previously by Towbin et al. in Proc. Natl. Acad. Sci. USA, 1979, 76, 4350-4354. The blots were treated with 5% (w/v) milk in PBST overnight at 4 °C. Subsequently, the protein blots were probed with a polyclonal antibody that was diluted 1:2,000 in PBST containing 5% (w/v) milk-powder and incubated for 2 hours (h) at room temperature. Further, the blots were washed six times, 5 min each at room temperature under gentle shaking. Subsequently, the blots were incubated in HRP-conjugated goat anti-rabbit IgG antibody for 1 h at room temperature diluted 1: 10,000 in PBST containing 5% (w/v) milk-powder. After incubation, the blots were washed six times with PBST, 5 min each at room temperature under gentle shaking. Finally, the indirectly bound HRP was detected by its enzymatic activity using luminol as substrate in the presence of hydrogen peroxide, as described by Mruk and Cheng in Spermatogenesis, 2011, 1, 121-122.

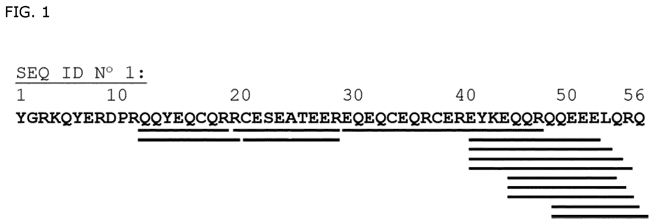

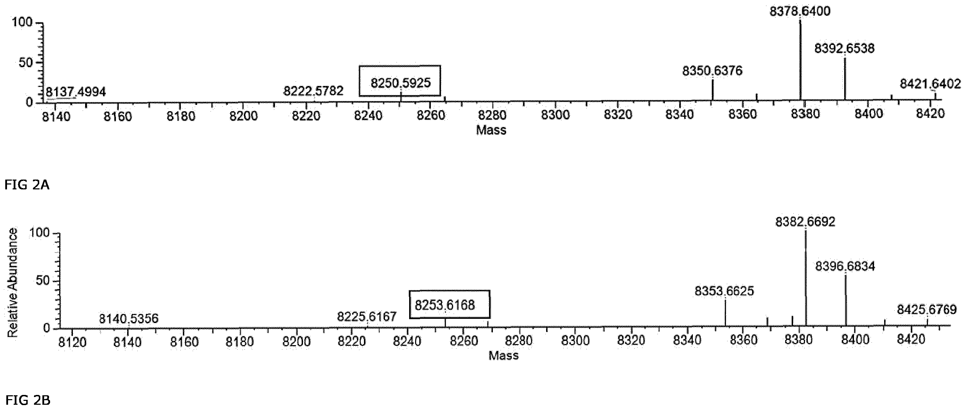

**Results:** Recombinant peptide of SEQ ID N° 1 was separated by SDS polyacrylamide gel electrophoresis, excised from the gel, and subjected to trypsin digestion resulting in a MALDI-TOF-MS peptide finger print depicted in **Figure 1****.** In both the reduced and non-reduced sample containing the peptides according to SEQ ID N° 1, masses of 8.3 kDa were detected using intact mass fingerprinting. The LC-MS results of the sample according to SEQ ID N° 1 are shown in **Figure 2****.**

### Example 2: Synthesis of the peptide

The peptide of sequence identical to SEQ ID N° 1 can be chemically synthesized by Seramun Diagnostica GmbH (Heidesee, Germany).

### Example 3: Minimum inhibitory concentration (MIC) assays

MIC assays determine the lowest concentration at which a compound inhibits the growth of a microbial population. The antimicrobial potential of the peptide described herein was compared with the activity of, two other AMPs, TcAMP1 and TcAMP2 as discussed in WO1998027805, and antibiotics or fungicides. Comparative MIC assays were carried out against several bacterial and fungal species (Table 1).

Peptides according to SEQ ID N° 1, SEQ ID N° 4, and SEQ ID N° 5 were serially diluted in order to create a concentration sequence ranging from 0.39 µg/ml to 303 µg/ml.

*Pantoea, Staphylococcus,* and *Listeria* were cultured in Trypticase Soy Broth (TSY) medium consisting of1.7% casein peptone, 0.3 % soy peptone, 0.25% glucose, 0.5% NaCl, 0.25% K₂HPO₄ and optionally 1.5% agar. *Candida* cells were cultured in Yeast Extract Peptone Dextrose (YPD) broth medium consisting of1%yeast extract, 2% peptone, 2% glucose, and optionally 2% agar. *Ceratocystis, Botrytis, Leptosphaeria, Mycosphaerella, Sclerotinia,* and *Verticillium* were cultured in Potato Dextrose Agar (PDA) medium consisting of2.4% Difco pre-mix broth and optionally 1.5% agar.

For the MIC assay bacteria and fungi were grown on agar plates with their respective media. Pre-cultures were prepared by inoculating either single bacterial colonies or small pieces of fungi in 5 ml of the respective liquid medium. The optical density (OD) at 600 nm was measured after one to five days and adjusted to an OD₆₀₀ of 1. This cell suspension was used to prepare 1:500 dilutions, corresponding to approximately 2 x 10⁶ microbial cells per ml.

To evaluate MICs, 96-well plates were prepared with the test antimicrobial peptides. Each well contained 100 µl microbial cell culture, 90 µl culture medium, and 10 µl peptide or control at the corresponding dilution. The plates were incubated overnight (ON) at the optimal growth temperature for each tested organism. Results were visually evaluated and MICs determined based on growth or no-growth.

**Table 1. Organisms that were tested in MIC assays. Given are the species name, classification as well as the growth conditions. DSM refers to the ordering number at the German Collection of Microorganisms and Cell Cultures GmbH (DSMZ, Braunschweig, Germany). SF refers to organisms obtained from the Jena Microbial Resource Collection (JMRC, Jena, Germany).**

| Species | Ordering number | Classification | Growth medium | Growth temperature |
|---|---|---|---|---|
| *Pantoea agglomerans* | JUB-CC-3253 | Gram-negative bacterium | TSY | 37°C |
| *Staphylococcus aureus* MRSA | DSM 11729 | Gram-positive bacterium | TSY | 37°C |
| *Listeria monocytogenes* | DSM 102976 | Gram-positive bacterium | TSY | 37°C |
| *Candida albicans* | DSM 1386 | Yeast | YPD | 30°C |
| *Ceratosystis paradoxa* | DSMZ 63054 | Fungus | PDA | 18°C |
| *Botrytis cinerea* | DSMZ 877 | Fungus | PDA | 24°C |
| *Leptosphaeria maculans* | SF 70 | Fungus | PDA | 18°C |
| *Mycosphaerella pinodes* | DSMZ 62763 | Fungus | PDA | 30°C |
| *Sclerotinia sclerotiorum* | SF 79 | Fungus | PDA | 18°C |
| *Verticillium dahliae* | SF 76 | Fungus | PDA | 28°C |

MIC assays were conducted in at least two independent replicates and with different peptide starting concentrations. Pre-tests showed, that none of the conducted steps to purify the tested proteins impacted obtained MICs.

**Results:** The MIC values, obtained for the peptides according to SEQ ID N° 1, SEQ ID N° 4, and SEQ ID N° 5 are shown in Table 2. The peptide having a sequence according to SEQ ID N° 1 had inhibitory effects against all the tested fungal species. Moreover, the peptide of the invention was more efficient in inhibiting fungal growth at a lower concentration than the peptide of the prior art in most of the tested species.

**Table 2. Results of MIC assays with fungal species as tester organisms and the peptide of the invention (SEQ ID N° 1) and the prior art peptides (SEQ ID N° 4 and N° 5). Clotrimazole was used as a reference.**

| Fungus | Minimal inhibitory concentration (µg/ml) | | | |
|---|---|---|---|---|
| | Clotrimazole | SEQ ID N° 1 | SEQ ID N° 4 | SEQ ID N° 5 |
| *Candida albicans* | 3.1 | 3.85 | 13.1 | 12.75 |
| *Ceratosystis paradoxa* | 0.4 | 1.9 | 4.8 | 4.7 |
| *Botrytis cinerea* | 6.25 | 3.5 | 14.5 | 12.75 |
| *Leptosphaeria maculans* | 0.4 | 12.5 | 37.9 | 9.4 |
| *Mycosphaerella pinodes* | 3.1 | 2.8 | 13.12 | 10.68 |
| *Sclerotinia sclerotiorum* | 25 | 10.4 | 27.2 | 23.5 |
| *Verticillium dahliae* | 1.56 | 3.85 | 14.5 | 12.7 |

The MIC values obtained for the peptides according to SEQ ID N° 1, SEQ ID N° 4, and SEQ ID N° 5 against bacterial species are shown in Table 3. The peptide having a sequence according to SEQ ID N° 1 had inhibitory effects against all the tested fungal species. Moreover, the peptide of the invention was more efficient in inhibiting fungal growth at a lower concentration than the peptide of the prior art in almost all the tested species with only the exception of *Leptosphaeria maculans* that was most effectively inhibited by the peptide with SEQ ID N° 5.

**Table 3. Results of MIC with bacterial species as tester organisms and the peptide of the invention (SEQ ID N° 1) and prior art peptides (SEQ ID N° 4 and N° 5). Gentamycin and Streptomycin were used as references.**

| Bacteria | Minimal inhibitory concentration (µg/ml) | | | | |
|---|---|---|---|---|---|
| | Gentamycin | Streptomycin | SEQ ID N° 1 | SEQ ID N° 4 | **SEQ ID** N° 5 |
| *Staphylococcus aureus* | 0.8 | >125 | 3.2 | 13 | 13.6 |
| *Listeria monocytogenes* | 0.4 | 7.8 | 1.6 | 3.25 | 13.6 |
| *Pantoea agglomerans* | 1.56 | NA | 2.5 | 7.9 | 9.45 |

### SEQUENCES LISTING

**SEQ ID N° 1(peptide according to an embodiment of the invention)**
   YGRKQYERDPRQQYEQCQRRCESEATEEREQEQCEQRCEREYKEQQRQQEEELQRQ
**SEQ ID N° 2 (signal peptide according to an embodiment of the invention)**
   MVISKSPFIVLIFSLLLSFALLCSGVSA
**SEQ ID** N° **3 (peptide according to an embodiment of the invention)** 
MVISKSPFIVLIFSLLLSFALLCSGVSAYGRKQYERDPRQQYEQCQRRCESEATEEREQEQCE
QRCEREYKEQQRQQEEELQRQ
**SEQ ID** N°4 **(prior** art **peptide)**
   **YERDPRQQYEQCQRRCESEATEEREQEQCEQRCEREYKEQQRQQEEE**
**SEQ ID N°5 (prior art peptide)** 
LQRQYQQCQGRCQEQQQGQREQQQCQRKCWEQYKEQERGEHENYHNHKKNRSEEEEGQ
QR
**SEQ ID N° 6 (Forward_BamH1 forward DNA primer)**
   CGCGGATCCTATGGCAGAAAACAATAT
**SEQ ID N° 7 (Reverse_Kpn1 forward DNA primer)**
   GGTACCTCATTGCCTTTGAAGCTCTTCTTCTT
**SEQ ID N° 8 (HQ tag)**
   HQHQHQ
**SEQ ID N° 9 (HN tag)**
   HNHNHNHNHNHN
**SEQ ID N° 10 (HAT tag)**
   KDHLIHNVHKEEHAHAHNK
**SEQ ID N° 11 (ALFA tag)**
   SRLEEELRRRLTE
**SEQ ID N° 12 (Avi tag)**
   GLNDIFEAQKIEWHE
**SEQ ID N° 13 (C-tag)**
   EPEA
**SEQ ID N° 14 (Calmodulin-tag)**
   KRRWKKNFIAVSAANRFKKISSSGAL
**SEQ ID N° 15 (E-tag)**
   GAPVPYPDPLEPR
**SEQ ID N° 16 (FLAG-tag)**
   DYKDDDDK
**SEQ ID N° 17 (HA-tag)**
   YPYDVPDYA
**SEQ ID N° 18 (Myc-tag)**
   EQKLISEEDL
**SEQ ID N° 19 (NE-tag)**
   TKENPRSNQEESYDDNES
**SEQ ID N° 20 (Rho1D4 tag)**
   TETSQVAPA
**SEQ ID N° 21 (S-tag)**
   KETAAAKFERQHMDS
**SEQ ID N° 22 (SBP tag)**
   DEKTTGWRGGHVVEGLAGELEQLRARLEHHPQGQREP
**SEQ ID N° 23 (Strep-tag)**
   WSHPQFEK

## Claims

1. A peptide having a sequence according to SEQ ID N° 1 or that differs in maximally 3 amino acids from the sequence according to SEQ ID N° 1 and still having the antimicrobial activity of the peptide having a sequence according to SEQ ID No 1.

2. The peptide according to claim 1, wherein said peptide has a sequence according to SEQ ID N° 1.

3. The peptide according to any of the claims 1 to 2, wherein said peptide is fused to a signal peptide at its N terminus.

4. The peptide according to claim 3, wherein said signal peptide has at least 95% sequence identity with a sequence according to SEQ ID N° 2, or is a sequence according to SEQ ID N° 2.

5. The peptide according to any of the previous claims 1 to 4 wherein, said peptide has antibacterial and/or antifungal activity.

6. The peptide according to any of the previous claims 1 to 5, wherein said activity is against Gram-positive, Gram-negative bacteria and/or fungi, preferably hyphal fungi and yeasts.

7. The peptide according to any of the previous claims wherein, said peptide is derivable from or isolated from Theobroma cacao, preferably Theobroma cacao variety CCN-51.

8. The peptide according to any of previous claims, wherein said peptide is a recombinant or a synthetic peptide.

9. A composition comprising a peptide according to any of the claims and an excipient.

10. The composition according to claim 9, wherein said composition is a liquid, a semisolid, a solid, or in gaseous form and/or wherein said composition is in a dosage form of a tablet, capsule, powder, granulate, aerosol, paste, syrup, suspension, emulsion or solution.

11. Peptide according to any of the claims 1 to 8 or composition according to any of the claims 9 to 10 for therapeutic use.

12. Peptide according to any of the claims 1 to 8 or composition according to claims 9 to 10 for use in the treatment of bacterial and/or fungal infections in a subject in need thereof.

13. Peptide for use according to claims 11 to 12, wherein said subject is a human or animal.

14. Use of a peptide according to claims 1 to 8 or composition according to claim 9 to 10, in crop protection, as a feed or food additive, a preservative, as a decontaminant or in cosmetical applications.

15. Use according to claim 14, wherein said peptide or composition is used for controlling a pathogen in a plant, preferably in a crop.

16. Use according to claim 15, wherein said pathogen is selected from the group of Gram-positive bacteria, Gram-negative bacteria, and/or fungi, preferably hyphal fungi or yeasts.

17. Method for producing a peptide according to any of the claims 1 to 8, wherein said peptide is produced by peptide recombination techniques or by synthesis.

18. A vector comprising a coding sequence for a peptide having a sequence according to SEQ ID N° 1, and optionally fused to a sequence for an N terminal signal peptide, said signal peptide preferably has at least 95% sequence identity with a sequence according to SEQ ID N° 2 wherein said vector is designed to allow expression of said peptide in an expression system.

19. A transgenic plant expressing a peptide according to any of the claims 1 to 8.

20. A method of producing the transgenic plant of claim 19, wherein said method comprises the step of introducing a nucleic acid encoding for said peptide into a plant cell thereby producing a transformed plant cell expressing said peptide and regenerating the transformed plant cell into a transgenic plants.

21. A seed of a plant coated with the peptide or composition according any of the claims 1 to 10.

## Patentansprüche

1. Ein Peptid mit einer Sequenz gemäß SEQ ID Nr. 1 oder einer, die sich in maximal 3 Aminosäuren von der Sequenz gemäß SEQ ID Nr. 1 unterscheidet und dennoch die antimikrobielle Aktivität des Peptids mit einer Sequenz gemäß SEQ ID Nr. 1 aufweist.

2. Das Peptid nach Anspruch 1, wobei das Peptid eine Sequenz gemäß SEQ ID Nr. 1 aufweist.

3. Das Peptid nach einem der Ansprüche 1 bis 2, wobei das Peptid an seinem N-Terminus mit einem Signalpeptid verschmolzen ist.

4. Das Peptid nach Anspruch 3, wobei das Signalpeptid mindestens 95 % Sequenzidentität mit einer Sequenz gemäß SEQ ID Nr. 2 aufweist oder eine Sequenz gemäß SEQ ID Nr. 2 ist.

5. Das Peptid nach einem der vorhergehenden Ansprüche 1 bis 4, wobei das Peptid eine antibakterielle und/oder antimykotische Aktivität aufweist.

6. Das Peptid nach einem der vorhergehenden Ansprüche 1 bis 5, wobei sich die Aktivität gegen grampositive, gramnegative Bakterien und/oder Pilze, vorzugsweise Fadenpilze und Hefen, richtet.

7. Das Peptid nach einem der vorhergehenden Ansprüche, wobei das Peptid vom Kakaobaum (Theobroma cacao), vorzugsweise der Kakaobaumsorte CCN-51, ableitbar ist oder aus diesem isoliert wird.

8. Das Peptid nach einem der vorhergehenden Ansprüche, wobei das Peptid ein rekombinantes oder ein synthetisches Peptid ist.

9. Eine Zusammensetzung, ein Peptid nach einem der Ansprüche und einen Hilfsstoff umfassend.

10. Die Zusammensetzung nach Anspruch 9, wobei die Zusammensetzung eine Flüssigkeit, ein Halbfeststoff, ein Feststoff oder gasförmig ist und/oder wobei die Zusammensetzung in einer Darreichungsform einer Tablette, einer Kapsel, eines Pulvers, eines Granulats, eines Aerosols, einer Paste, eines Sirups, einer Suspension, einer Emulsion oder einer Lösung vorliegt.

11. Peptid nach einem der Ansprüche 1 bis 8 oder Zusammensetzung nach einem der Ansprüche 9 bis 10 zur therapeutischen Verwendung.

12. Peptid nach einem der Ansprüche 1 bis 8 oder Zusammensetzung nach einem der Ansprüche 9 bis 10 zur Verwendung bei der Behandlung von bakteriellen und/oder Pilzinfektionen in einem Subjekt, das dessen bedarf.

13. Peptid zur Verwendung nach den Ansprüchen 11 bis 12, wobei das Subjekt ein Mensch oder ein Tier ist.

14. Verwendung eines Peptids nach einem der Ansprüche 1 bis 8 oder einer Zusammensetzung nach einem der Ansprüche 9 bis 10 beim Pflanzenschutz, als Futter- oder Nahrungsmittelzusatz, als Konservierungsmittel, als Dekontaminationsmittel oder in kosmetischen Anwendungen.

15. Verwendung nach Anspruch 14, wobei das Peptid oder die Zusammensetzung verwendet wird, um ein Pathogen in einer Pflanze zu kontrollieren, vorzugsweise bei einer Feldfrucht.

16. Verwendung nach Anspruch 15, wobei das Pathogen aus der Gruppe grampositiver Bakterien, gramnegativer Bakterien und/oder Pilzen ausgewählt ist, vorzugsweise Fadenpilze oder Hefen.

17. Verfahren zur Herstellung eines Peptids nach einem der Ansprüche 1 bis 8, wobei das Peptid durch Peptidrekombinationstechniken oder durch Synthese produziert wird.

18. Ein Vektor, eine Codierungssequenz für ein Peptid mit einer Sequenz gemäß SEQ ID Nr. 1 und optional mit einer Sequenz für ein N-Terminus-Signalpeptid verschmolzen umfassend, wobei das Signalpeptid vorzugsweise mindestens 95 % Sequenzidentität mit einer Sequenz gemäß SEQ ID Nr. 2 aufweist, wobei der Vektor dafür gestaltet ist, die Expression des Peptids in einem Expressionssystem zu ermöglichen.

19. Eine transgene Pflanze, ein Peptid nach einem der Ansprüche 1 bis 8 exprimierend.

20. Ein Verfahren zum Produzieren der transgenen Pflanze nach Anspruch 19, wobei das Verfahren den Schritt des Einbringens einer Nukleinsäurecodierung für das Peptid in eine Pflanzenzelle, wodurch eine transformierte Pflanzenzelle produziert wird, die das Peptid exprimiert, und des Regenerierens der transformierten Pflanzenzelle in eine transgene Pflanze umfasst.

21. Ein Saatgut einer Pflanze, das mit dem Peptid oder der Zusammensetzung nach einem der Ansprüche 1 bis 10 überzogen ist.

## Revendications

1. Un peptide ayant une séquence selon SEQ ID N° 1 ou qui diffère au maximum de 3 acides aminés de la séquence selon SEQ ID N° 1 et possédant toujours l'activité antimicrobienne du peptide ayant une séquence selon SEQ ID N° 1.

2. Le peptide selon la revendication 1, dans lequel ledit peptide a une séquence selon SEQ ID N° 1.

3. Le peptide selon l'une quelconque des revendications 1 à 2, dans lequel ledit peptide est fusionné à un peptide signal à son extrémité N-terminale.

4. Le peptide selon la revendication 3, dans lequel ledit peptide signal a au moins 95 % d'identité de séquence avec une séquence selon SEQ ID N° 2, ou est une séquence selon SEQ ID N° 2.

5. Le peptide selon l'une quelconque des revendications précédentes 1 à 4, dans lequel ledit peptide a une activité antibactérienne et/ou antifongique.

6. Le peptide selon l'une quelconque des revendications précédentes 1 à 5, dans lequel ladite activité est contre les bactéries à Gram-positif, à Gram-négatif, et/ou les champignons, de préférence les champignons à hyphes et les levures.

7. Le peptide selon l'une quelconque des revendications précédentes, dans lequel ledit peptide peut être dérivé ou est isolé du Theobroma cacao, de préférence de la variété Theobroma cacao CCN-51.

8. Le peptide selon l'une quelconque des revendications précédentes, dans lequel ledit peptide est un peptide recombinant ou synthétique.

9. Une composition comprenant un peptide selon l'une quelconque des revendications et un excipient.

10. La composition selon la revendication 9, dans laquelle ladite composition est un liquide, un semi-solide, un solide, ou sous forme gazeuse et/ou dans laquelle ladite composition se présente sous une forme posologique d'un comprimé, d'une capsule, d'une poudre, d'un granulé, d'un aérosol, d'une pâte, d'un sirop, d'une suspension, d'une émulsion ou d'une solution.

11. Peptide selon l'une quelconque des revendications 1 à 8 ou composition selon l'une quelconque des revendications 9 à 10 pour un usage thérapeutique.

12. Peptide selon l'une quelconque des revendications 1 à 8 ou composition selon les revendications 9 à 10 destinés à être utilisés dans le traitement des infections bactériennes et/ou fongiques chez un sujet en ayant besoin.

13. Peptide destiné à l'utilisation selon les revendications 11 à 12, dans lequel ledit sujet est un humain ou un animal.

14. Utilisation d'un peptide selon les revendications 1 à 8 ou d'une composition selon la revendication 9 à 10, dans la protection des cultures, comme aliment ou additif alimentaire, comme conservateur, comme décontaminant ou dans des applications cosmétiques.

15. Utilisation selon la revendication 14, dans laquelle ledit peptide ou ladite composition est utilisé(e) pour lutter contre un agent pathogène dans une plante, de préférence dans une culture.

16. Utilisation selon la revendication 15, dans laquelle ledit agent pathogène est choisi dans le groupe constitué par les bactéries à Gram positif, les bactéries à Gram négatif et/ou les champignons, de préférence les champignons à hyphes ou les levures.

17. Procédé de production d'un peptide selon l'une quelconque des revendications 1 à 8, dans lequel ledit peptide est produit par des techniques de recombinaison peptidique ou par synthèse.

18. Un vecteur comprenant une séquence codante pour un peptide ayant une séquence selon SEQ ID N° 1, et éventuellement fusionné à une séquence pour un peptide signal N-terminal, ledit peptide signal a de préférence au moins 95 % d'identité de séquence avec une séquence selon SEQ ID N° 2, dans lequel ledit vecteur est conçu pour permettre l'expression dudit peptide dans un système d'expression.

19. Une plante transgénique exprimant un peptide selon l'une quelconque des revendications 1 à 8.

20. Un procédé de production de la plante transgénique selon la revendication 19, dans lequel ledit procédé comprend l'étape consistant à introduire un acide nucléique codant pour ledit peptide dans une cellule végétale, produisant ainsi une cellule végétale transformée exprimant ledit peptide et régénérant la cellule végétale transformée dans une plante transgénique.

21. Une graine d'une plante enrobée du peptide ou de la composition selon l'une quelconque des revendications 1 à 10.
